# EUROPÄISCHE PATENTSCHRIFT

(11) **EP 1 966 083 B1**
(45) Veröffentlichungstag und Bekanntmachung des Hinweises auf die Patenterteilung: **18.01.2012**
(21) Anmeldenummer: 06830062.3
(22) Anmeldetag: 21.11.2006
(51) Int. Cl.: C01B 21/22, B01D 53/14, C07C 45/27

(54) **VERFAHREN ZUR ISOLIERUNG VON N2O**
METHOD FOR ISOLATING N2O
PROCEDE D'ISOLEMENT DE N2O

(30) Priorität: 22.11.2005 DE 102005055588
(43) Veröffentlichungstag der Anmeldung: 10.09.2008
(73) Patentinhaber: BASF SE, 67056 Ludwigshafen (DE)
(72) Erfinder: TELES, Joaquim Henrique, 67166 Otterstadt (DE); RÖSSLER, Beatrice, 68256 Weisenheim am Sand (DE); BAUMANN, Dieter, 67346 Speyer (DE)
(74) Vertreter: Altmann, Andreas
(86) Internationale Anmeldenummer: PCT/EP2006/068714
(87) Internationale Veröffentlichungsnummer: WO 2007/060160

(56) Entgegenhaltungen:
- WO-A2-2005/030690
- DE-A1- 2 040 219
- GB-A- 649 680
- US-A- 4 844 715
- US-A- 6 080 226
- US-A1- 2005 203 316

## Beschreibung

Die vorliegende Erfindung betrifft ein Verfahren zur Reinigung eines Gasgemisches G-0 enthaltend Distickstoffmonoxid.

Aus dem Stand der Technik sind verschiedene Herstellungsverfahren für Distickstoffmonoxid bekannt. Es ist ebenfalls bekannt, dass Distickstoffmonoxid beispielsweise als Oxidationsmittel für Olefine eingesetzt werden kann.

So offenbart die WO 98/25698 ein Verfahren zur Herstellung von Distickstoffmonoxid durch katalytische Partialoxidation von NH₃ mit Sauerstoff. Dabei wird gemäß der WO 98/25698 ein Katalysator aus Manganoxid, Bismutoxid und Aluminiumoxid eingesetzt, der mit hoher Selektivität zu Distickstoffmonoxid führt. Ein ähnliches Katalysatorsystem wird auch in einer wissenschaftlichen Arbeit näher beschrieben (Noskov et al., Chem. Eng. J. 91 (2003) 235-242). Gemäß der US 5,849,257 wird ebenfalls ein Verfahren zur Herstellung von Distickstoffmonoxid durch Oxidation von Ammoniak offenbart. Dabei findet die Oxidation in Gegenwart eines Kupfer-Manganoxid Katalysators statt.

Gemäß dem in der WO 00/01654 offenbarten Verfahren wird Distickstoffmonoxid hergestellt, indem ein Gasstrom enthaltend NOₓ und Ammoniak reduziert wird.

Die Oxidation einer olefinischen Verbindung zu einem Aldehyd oder einem Keton mittels Distickstoffmonoxid ist beispielsweise beschrieben in der GB 649,680 oder der dazu äquivalenten US 2,636,898. In beiden Schriften wird ganz allgemein offenbart, dass die Oxidation prinzipiell in Anwesenheit eines geeigneten Oxidationskatalysators erfolgen kann.

In den neueren wissenschaftlichen Artikeln von G. L. Panov et al., "Non-Catalytic Liquid Phase Oxidation of Alkenes with Nitrous Oxide. 1. Oxidation of Cyclohexene to Cyclohexanone", React. Kinet. Catal. Lett. Vol. 76, No. 2 (2002) S. 401-405, und K. A. Dubkov et al., "Non-Catalytic Liquid Phase Oxidation of Alkenes with Nitrous Oxide. 2. Oxidation of Cyclopentene to Cyclopentanone", React. Kinet. Catal. Lett. Vol. 77, No. 1 (2002) S. 197-205 werden ebenfalls Oxidationen von olefinischen Verbindungen mit Distickstoffmonoxid beschrieben. Auch ein wissenschaftlicher Artikel "Liquid Phase Oxidation of Alkenes with Nitrous Oxide to Carbonyl Compounds" von E. V. Starokon et al. in Adv. Synth. Catal. 2004, 346, 268 - 274 beinhaltet eine mechanistische Studie der Oxidation von Alkenen mit Distickstoffmonoxid in flüssiger Phase.

Die Synthese von Carbonylverbindungen aus Alkenen mit Distickstoffmonoxid wird auch in verschiedenen internationalen Patentanmeldungen beschrieben. So offenbart die WO 03/078370 ein Verfahren zur Herstellung von Carbonylverbindungen aus aliphatischen Alkenen mit Distickstoffmonoxid. Die Umsetzung wird bei Temperaturen im Bereich von 20 bis 350°C und Drücken von 0,01 bis 100 atm durchgeführt. Die WO 03/078374 offenbart ein entsprechendes Verfahren zur Herstellung von Cyclohexanon. Gemäß der WO 03/078372 werden cyclische Ketone mit 4 bis 5 Atomen hergestellt. Gemäß der WO 03/078375 werden unter diesen Verfahrensbedingungen cyclische Ketone aus cyclischen Alkenen mit 7 bis 20 C-Atomen hergestellt. WO03/078371 offenbart ein Verfahren zur Herstellung substituierter Ketone aus substituierten Alkenen. WO 041000777 offenbart ein Verfahren zur Umsetzung von Di- und Polyalkenen mit Distickstoffmonoxid zu den entsprechenden Carbonylverbindungen. Die Reinigung von Distickstoffmonoxid wird in diesen Schriften nicht erwähnt.

Es ist ebenfalls bekannt, dass Abgasströme enthaltend Distickstoffmonoxid für weitere Umsetzungen eingesetzt werden können. Distickstoffmonoxid fällt als unerwünschtes Nebenprodukt bei verschiedenen chemischen Prozessen an, insbesondere bei Oxidationen mit Salpetersäure und dort ganz besonders bei der Oxidation von Cyclohexanon und/oder Cyclohexanol zu Adipinsäure. Andere Beispiele für Verfahren, bei denen Distickstoffmonoxid als unerwünschtes Nebenprodukt anfällt, sind die Oxidation von Cyclododecanon und/oder Cyclododecanol mit Salpetersäure zu Dodecandicarbonsäure und die Partialoxidation von NH₃ zu NO.

So offenbaren die WO 2005/030690, die WO 2005/030689 und die WO 2004/096745 Verfahren zur Oxidation von Olefinen mit Distickstoffmonoxid, nämlich die Oxidation von Cyclododecatrien, von Cyclododecen und von Cyclopenten. Alle drei Anmeldungen offenbaren, dass neben anderen Disfickstoffmonoxidquellen auch Abgasströme eingesetzt werden können, die beispielsweise durch destillative Methoden aufgereinigt werden können, bevor sie als Oxidationsmittel eingesetzt werden.

In der GB 649,680 wird ein Verfahren zur Oxidation von Olefinen mittels Distickstoffmonoxid offenbart. Dabei wird auch offenbart, dass es sich bei den Olefinen um zyklische Olefine handeln kann, die mittels Distickstoffmonoxid zu cyclischen Ketonen oxidiert werden.

Sowohl bei der Herstellung von Distickstoffmonoxid als auch bei der Verwendung von Abgasströmen fällt N₂O zunächst als verdünntes gasförmiges Gemisch mit anderen Komponenten an. Diese Komponenten lassen sich unterteilen in solche, die für spezielle Anwendungen störend wirken, und solche, die sich inert verhalten. Für den Einsatz als Oxidationsmittel sind als solche störend wirkenden Gase unter anderem NOₓ oder beispielsweise Sauerstoff zu nennen. Der Begriff "NOₓ", wie er im Rahmen der vorliegenden Erfindung verstanden wird, bezeichnet sämtliche Verbindungen NₐO_{b}, wobei a 1 oder 2 ist und b eine Zahl von 1 bis 6, außer N₂O. Statt dem Begriff "NOₓ" wird im Rahmen der vorliegenden Erfindung auch der Begriff "Stickoxide" verwendet. Als störende Nebenkomponenten sind auch NH₃ und organischen Säuren zu nennen.

Für spezielle Anwendungen ist es nötig, das eingesetzte Distickstoffmonoxid vor der Umsetzung zu reinigen. Beispielsweise für die Verwendung von Distickstoffmonoxid als Oxidationsmittel ist es nötig, störende Nebenkomponenten wie Sauerstoff oder Stickoxide NOₓ abzutrennen.

Verfahren zur Abtrennung von NOₓ sind grundsätzlich aus dem Stand der Technik bekannt. Eine Übersicht gibt beispielsweise M. Thiemann et. al in Ullmann's Encyclopedia, 6th Edition, 2000, Electronic Edition, Kapitel "Nitric Acid, Nitrous Acid, and Nitrogen Oxides", Abschnitt 1.4.2.3.

Die Anmeldung WO 00/73202 beschreibt eine Methode, wie man NOₓ und O₂ aus einem N₂O-haltigen Gasstrom entfernen kann. Das NOₓ wird durch katalytische Reduktion mit NH₃ entfernt und Sauerstoff durch katalytische Reduktion mit Wasserstoff oder anderen Reduktionsmitteln. Diese Methode hat aber den Nachteil, dass das Produkt mit NH₃ kontaminiert wird. Eine starke Abreicherung von Sauerstoff, beispielsweise auf über 90% der ursprünglichen Menge, ist nur möglich, wenn ein Verlust an N₂O von beispielsweise 3 bis 5% der ursprünglich enthaltenen Menge in Kauf genommen wird.

Für spezielle Anwendungen kann es notwendig sein, auch die inerten Verbindungen abzutrennen, da sie die gewünschte Umsetzung mit N₂O durch Verdünnung verlangsamen können. Der Begriff "Inertgas", wie er im Rahmen der vorliegenden Erfindung verwendet wird, bezeichnet ein Gas, das sich hinsichtlich der Umsetzung von N₂O mit einem Olefin inert verhält, also unter den Bedingungen der Umsetzung von Olefinen mit N₂O weder mit den Olefinen noch mit N₂O reagieren. Als Inertgase sind beispielsweise Stickstoff, Kohlendioxid, Kohlenmonoxid, Wasserstoff, Argon, Methan, Ethan und Propan zu nennen. Die Inertgase können aber die Raum-ZeitAusbeute senken, so dass eine Abreicherung ebenfalls vorteilhaft sein kann. Es kann aber ebenfalls vorteilhaft sein, ein Gasgemisch zu erhalten, das noch Inertgase enthält, und dann direkt in eine weitere Umsetzung eingesetzt werden kann.

In DE 27 32 267 A1 wird beispielsweise ein Verfahren zur Reinigung von Distickstoffmonoxid offenbart, wobei zunächst Stickstoffoxid, Stickstoffdioxid, Kohlenstoffdioxid und Wasser abgetrennt werden und anschließend das Gasgemisch durch Kompression auf 40 bis 300 bar und Kühlung aus 0 bis -88°C verflüssigt wird. Aus diesem verflüssigten Gasgemisch wird dann Distickstoffmonoxid abgetrennt. Diese Methode erreicht zwar eine Reinigung und Aufkonzentrierung des N₂O, ist aber aufgrund des geforderten hohen Drucks (60 bar), der tiefen Temperaturen (-85°C) und den damit verbundenen hohen Investitionen, wirtschaftlich unattraktiv.

In US 4,177,645 wird ein Verfahren zur Abtrennung von Distickstoffmonoxid aus Abgasströmen offenbart, das ebenfalls eine Vorreinigung und eine Tieftemperaturdestillation umfasst. Die Anmeldung EP 1 076 217 A1 beschreibt ebenfalls eine Methode zur Entfernung von leichtsiedenden Verunreinigungen aus N₂O durch Tieftemperaturdestillation.

Auch US 6,505,482, US 6,370,911 und US 6,387,161 offenbaren Verfahren zur Reinigung von Distickstoffmonoxid, bei dem jeweils eine Tieftemperaturdestillation in einer speziellen Anlage durchgeführt wird.

Eine Tieftemperaturdestillabon erfordert durch die hohen Drücke und tiefen Temperaturen jedoch apparativ einen hohen Aufwand, der die Reinigung des Distickstoffmonoxids mit einem derartigen Verfahren aufwändig und kostenintensiv macht. Besonders störend ist hierbei die Tatsache, dass bei Normaldruck der Schmelzpunkt von N₂O nur 3K unterhalb des Siedepunkts liegt. Daher müssen hohe Drücke angewandt werden.

Die US 6,080,226 offenbart ein Verfahren zur Reinigung von Distickstoffmonoxid durch Druckwechselabsorption. US 4,844,715 offenbart ein Verfahren zur Auftrennung von Edelgasen. Dabei wird ein Absorption-Desorptionsverfahren eingesetzt. Hierbei werden in einem Schritt das zu reinigende Edelgas und Distickstoffmonoxid getrennt, wobei Distickstoffmonoxid im Absorptionsmittel verbleibt.

DE20 40 219 offenbart ein Herstellungsverfahren für Distickstoffmonoxid, wobei das erhaltene Distickstoffmonoxid nach der Synthese konzentriert und gereinigt wird. Dabei wird gemäß der DE20 40 219 zunächst Distickstoffmonoxid durch Oxidation von Ammoniak hergestellt. Das hergestellte Distickstoffmonoxid wird gereinigt, indem die oxidierten Gase separiert und durch Absorption unter hohem Druck, der eine Desorption unter vermindertem Druck folgt, konzentriert. Nebenkomponenten werden beispielsweise durch Behandlung mit einer Alkalilösung entfernt. Als Lösungsmittel für die Absorption des Gasgemisches wird gemäß DT 20 40 219 Wasser verwendet,

Mit dem in DE20 40 219 offenbarten Verfahren ist eine Trennung der verschiedenen Stickoxide möglich, das Verfahren erfordert jedoch auf Grund der vergleichsweise geringen Löslichkeit von N₂O in Wasser den Einsatz von großen Lösungsmittelmengen und/oder hoher Drücke für die Absorption.

In DE 10 2004 046167.8 wird ein Verfahren zur Reinigung eines Gasgemisches enthaltend Distickstoffmonoxid offenbart, das mindestens eine Absorption des Gasgemisches in einem organischen Lösungsmittel und anschließende Desorption des Gasgemisches aus dem beladenen organischen Lösungsmittel sowie das Einstellen des Gehalts an Stickoxiden NOₓ in dem Gasgemisch auf höchstens 0,5 Vol.-%, bezogen auf das Gesamtvolumen des Gasgemisches umfasst. In DE 10 2004 046167.8 wird auch offenbart, dass das Verfahren mehrere Absorptions- und Desorptionsschritte umfassen kann. In DE 10 2004 046167.8 werden nur organische Lösungsmittel als Absorptionsmedium offenbart.

Ausgehend von diesem Stand der Technik lag eine Aufgabe der vorliegenden Erfindung darin, ein sicherheitstechnisch unbedenkliches Verfahren bereitzustellen, mit dem Distickstoffmonoxid-haltige Ströme effektiv und kostengünstig gereinigt und aufkonzentriert werden können. Derart aufgereinigtes Distickstoffmonoxid wird insbesondere als Oxidationsmittel benötigt.

Eine weitere Aufgabe der vorliegenden Erfindung war es, Verfahren zur Herstellung von Gasgemischen bereitzustellen, die ohne weitere Behandlung oder Zusatz anderer Inertisierungsmittel als Oxidationsmittel eingesetzt werden können.

Erfindungsgemäß wird diese Aufgabe gelöst durch ein Verfahren zur Reinigung eines Gasgemisches G-0 enthaltend Distickstoffmonoxid, mindestens umfassend die folgenden Schritte:
- A1: Absorption des Gasgemisches G-0 in einem organischen Lösungsmittel
- A2: Desorption eines Gasgemisches G-1 aus dem beladenen organischen Lösungsmittel
- B1: Absorption des Gasgemisches G-1 in Wasser
- B2: Desorption eines Gasgemisches G-2 aus dem beladenen Wasser.

Das erfindungsgemäße Verfahren hat unter anderem den Vorteil, dass neben den störenden Komponenten teilweise auch die inerten Komponenten abgetrennt werden. Das erfindungsgemäß gereinigte Distickstoffmonoxid wird also gleichzeitig aufkonzentriert.

Erfindungsgemäß weist das Gasgemisch G-1 einen höheren Gehalt an Distickstoffmonoxid auf als das Gasgemisch G-0. Das Gasgemisch G-2 weist erfindungsgemäß wiederum einen höheren Gehalt an Distickstoffmonoxid auf als das Gasgemisch G-1.

Dabei hat das erfindungsgemäße Verfahren darüber hinaus den Vorteil, dass bei der zweiten Absorption, also bei einem höheren Gehalt an Distickstoffmonoxid, Wasser als Absorptionsmittel eingesetzt wird. Damit wird vermieden, dass Distickstoffmonoxid als starkes Oxidationsmittel in höheren Konzentrationen mit einem organischen Lösungsmittel in Kontakt gebracht wird, was zu einem hohen apparativen Aufwand und hohen Kosten führen würde. Durch die erfindungsgemäße Verwendung von Wasser als Lösungsmittel in der zweiten Absorption und Desorption gemäß Schritt B1 und B2 wird darüber hinaus vermieden, dass das Gasgemisch G-2 mit organischem Lösungsmittel kontaminiert wird, was zu weiteren Reinigungsschritten führen könnte.

Die Verwendung einer zweistufigen Absorption/Desorption mit einem organischen Lösungsmittel gemäß Schritt A1 und A2 und Wasser gemäß Schritt B1 und B2 hat insbesondere den Vorteil, dass in der ersten Stufe die hohe Löslichkeit von N₂O in organischen Lösungsmitteln genutzt wird, um mit kleineren Apparaten und geringere Kreisläufen hohe Aufkonzentrierungsfaktoren zu erzielen. Das Gasgemisch G-1 weist nach der ersten Absorption/Desorption bereits eine N₂O Konzentration auf, die aus sicherheitstechnischen Gründen eine Verwendung von Wasser als Lösungsmittel bei der Absorption/Desorption vorteilhaft macht. Die höhere N₂O Konzentration in der zweiten Stufe ermöglicht trotzdem die Verwendung kleinerer Apparate.

Derart gereinigtes Distickstoffmonoxid kann insbesondere in flüssiger Form vorteilhaft als Oxidationsmittel eingesetzt werden. Vorteilhafterweise kann das Gasgemisch G-2 neben Distickstoffmonoxid auch Kohlenstoffdioxid enthalten. CO₂ wirkt inertisierend und gewährleistet einen sicherheitstechnisch unbedenklichen Betrieb bei der Aufbereitung und insbesondere bei der Lagerung und weiteren Verwendung des Gasgemischs G-2 enthaltend Distickstoffmonoxid. Es wurde gefunden, dass bei der Anwesenheit von CO₂ als Inertgas in Gasgemischen enthaltend N₂O im Vergleich zu anderen Inertgasen deutlich geringere Mengen Kohlenstoffdioxid benötigt werden, um die Selbstzerfallfähigkeit von Distickstoffmonoxid zu unterbinden. Damit reichen geringen Mengen an CO₂ zur Inertisierung aus.

Erfindungsgemäß kann das eingesetzte Gasgemisch G-0 enthaltend Distickstoffmonoxid grundsätzlich aus jeder beliebigen Quelle stammen.

Der Begriff "Gasgemisch", wie er im Rahmen der vorliegenden Erfindung verwendet wird, bezeichnet ein Gemisch aus zwei oder mehr Verbindungen, die sich bei umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig, und wird im Rahmen der vorliegenden Erfindung weiter als Gasgemisch bezeichnet.

Wird ein Gasgemisch G-0 eingesetzt, so ist dessen Gehalt an Distickstoffmonoxid im Wesentlichen beliebig, solange gewährleistet ist, dass die erfindungsgemäße Reinigung möglich ist.

Die N₂O-haltigen Gasgemische, die für dieses Verfahren als Gasgemisch G-0 eingesetzt werden, haben in der Regel einen N₂O-Gehalt zwischen 2 und 80 Vol.-% N₂O. Es enthält ferner beispielsweise 2 bis 21 Vol.% O₂ und bis zu 30 Vol.% NOₓ als unerwünschte Komponenten. Ferner kann es noch in wechselnden Mengen N₂, H₂, CO₂, CO, H₂O, NH₃ enthalten, in Spuren können auch noch Salpetersäure und organische Verbindungen enthalten sein.

Im Rahmen der vorliegenden Erfindung wird die Zusammensetzung der Gasgemische oder der verflüssigten Gasgemische in Vol.-% angegeben. Dabei beziehen sich die Angaben auf die Zusammensetzung der Gasgemische bei Umgebungsdruck und Umgebungstemperatur.

Gemäß einer bevorzugten Ausführungsform des erfindungsgemäßen Verfahrens wird ein mindestens 3 Vol.-% Distickstoffmonoxid enthaltendes Gasgemisch G-0 eingesetzt, wobei wiederum bevorzugt Gemische mit einem Distickstoffmonoxid-Gehalt im Bereich von 4 bis 60 Vol.-%, weiter bevorzugt im Bereich von 5 bis 25 Vol.-% und insbesondere bevorzugt im Bereich von 8 bis 14 Vol.-% eingesetzt werden.

Grundsätzlich kann die Zusammensetzung der Gemische im Rahmen der vorliegenden Erfindung auf jede dem Fachmann bekannte Weise bestimmt werden. Die Zusammensetzung der Gasgemische wird im Rahmen der vorliegenden Erfindung vorzugsweise gaschromatographisch bestimmt. Sie kann jedoch auch mittels UV-Spektroskopie, IR-Spektroskopie oder nasschemisch bestimmt werden.

Gemäß einer bevorzugten Ausführungsform der vorliegenden Erfindung ist das Gasgemisch G-0 enthaltend Distickstoffmonoxid mindestens ein Distickstoffmonoxid enthaltendes Abgas eines chemischen Verfahrens. Im Rahmen der vorliegenden Erfindung sind auch Ausführungsformen umfasst, bei denen mindestens zwei Stickstoffmonoxid enthaltende Abgase einer einzigen Anlage als Gasgemisch enthaltend Distickstoffmonoxid dienen. Ebenso sind Ausführungsformen umfasst, bei denen mindestens ein Distickstoffmonoxid enthaltendes Abgas einer Anlage und mindestens ein weiteres Distickstoffmonoxid enthaltendes Abgas mindestens einer weiteren Anlage als Gasgemisch enthaltend Distickstoffmonoxid dienen.

Demgemäß betrifft die vorliegende Erfindung auch ein wie oben beschriebenes Verfahren, wobei das Gasgemisch enthaltend Distickstoffmonoxid mindestens ein Distickstoffmonoxid enthaltendes Abgas mindestens eines industriellen Verfahrens ist.

Der Begriff Gasgemisch enthaltend Distickstoffmonoxid" bezeichnet im Rahmen der vorliegende Erfindung sowohl Ausführungsformen, in denen das genannte Abgas in unmodifizierter Form dem erfindungsgemäßen Reinigungsverfahren unterworfen wird, als auch Ausführungsformen, in denen mindestens eines der genannten Abgase einer Modifikation unterworfen wird.

Der Begriff "Modifikation", wie er in diesem Zusammenhang im Rahmen der vorliegenden Erfindung verwendet wird, bezeichnet jedes geeignete Verfahren, mit dem die chemische Zusammensetzung eines Gasgemisches verändert wird. Demgemäß umfasst der Begriff "Modifikation" unter anderem Ausführungsformen, in denen ein Distickstoffmonoxid enthaltendes Abgas bezüglich des Distickstoffmonoxid-Gehaltes gemäß mindestens eines geeigneten Verfahrens aufkonzentriert wird. Vorzugsweise wird das Abgas keiner Modifikation unterworfen.

Gemäß einer weiteren Ausführungsform kann die chemische Zusammensetzung eines Abgases auch durch Zusatz von reinem Distickstoffmonoxid zu dem Abgas verändert werden.

Das eingesetzte Gasgemisch G-0 enthaltend N₂O kann beispielsweise ein Abgas aus einem industriellen Verfahren sein. Vorzugsweise stammt es aus einem Abgas einer Anlage zur Herstellung von Carbonsäuren durch Oxidation von Alkoholen oder Ketonen mit Salpetersäure, wie z.B. aus einer Adipinsäure- oder Dodecandicarbonsäure-Anlage, aus dem Abgas einer Salpetersäure-Anlage die die obigen Abgasströme als Edukt einsetzt, aus dem Abgas einer Anlage zur Partialoxidation von NH₃ oder aus dem Abgas einer Anlage die die darin erzeugten Gasgemische einsetzt, wie z.B. einer Hydroxylamin-Anlage.

Erfindungsgemäß kann auch ein Gemisch verschiedener Abgase eingesetzt werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandicarbonsäureanlage, einer Hydroxylaminanlage und/oder einer Salpetersäureanlage, wobei letztere wiederum bevorzugt mit mindestens einem Abgas einer Adipinsäureanlage, einer Dodecandicarbonsäureanlage oder einer Hydroxylaminanlage betrieben wird.

Gemäß einer bevorzugten Ausführungsform wird der Abgasstrom einer Adipinsäureanlage eingesetzt, bei der durch Oxidation von Cyclohexanol/Cyclohexanon-Gemischen mit Salpetersäure pro Mol gebildeter Adipinsäure im Allgemeinen 0,8 bis 1,0 mol N₂O gebildet werden. Wie beispielsweise in A. K. Uriarte et al., Stud. Surf. Sci. Catal. 130 (2000) S. 743-748 beschrieben, enthalten die Abgase von Adipinsäureanlagen in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Bei der obenstehend erwähnten Dodecandicarbonsäureanlage handelt es sich um den im Wesentlichen identischen Anlagentyp.

Eine beispielsweise typische Zusammensetzung eines Abgases einer Adipinsäureanlage oder einer Dodecandicarbonsäureanlage ist in der folgenden Tabelle wiedergegeben:

| Komponente | Konzentrationen / Gew.-% |
|---|---|
| NOₓ | 19 - 25 |
| N₂O | 20 - 28 |
| N₂ | 30 - 40 |
| O₂ | 7 - 10 |
| CO₂ | 2 - 3 |
| H₂O | - 7 |

Der Abgasstrom einer Adipinsäureanlage oder einer Dodecandicarbonsäureanlage kann direkt in dem erfindungsgemäßen Verfahren eingesetzt werden.

Gemäß einer ebenfalls bevorzugten Ausführungsform wird der Abgasstrom einer Salpetersäureanlage eingesetzt, die ganz oder teilweise mit Distickstoffmonoxid und Stickoxide enthaltenden Abgasen aus anderen Verfahren gespeist wird. In derartigen Salpetersäureanlagen werden Stickoxide adsorbiert und zum größten Teil zu Salpetersäure umgesetzt, während Distickstoffmonoxid nicht umgesetzt wird. Beispielsweise kann eine derartige Salpetersäureanlage durch Stickoxide, die durch gezielte Verbrennung von Ammoniak hergestellt werden, und durch Abgase einer Adipinsäureanlage und/oder durch Abgase einer Dodecandicarbonsäureanlage gespeist werden. Ebenso ist es möglich, eine derartige Salpetersäureanlage allein durch Abgase einer Adipinsäureanlage und/oder durch Abgase einer Dodecandicarbonsäureanlage zu speisen.

Die Abgase von derartigen Salpetersäureanlagen enthalten grundsätzlich in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Eine beispielsweise typische Zusammensetzung eines Abgases einer derartigen Salpetersäureanlage ist in der folgenden Tabelle wiedergegeben:

| Komponente | Konzentrationen / Gew.-% |
|---|---|
| NOₓ | < 0,1 |
| N₂O | 4-36 |
| N₂ | 57 - 86 |
| O₂ | 3 - 9 |
| CO₂ | 1 - 4 |
| H₂O | - 0,6 |

Der Abgasstrom einer Salpetersäureanlage kann direkt in dem erfindungsgemäßen Verfahren eingesetzt werden.

Gemäß einer ebenfalls bevorzugten Ausführungsform des efindungsgemäßen Verfahrens wird der Abgasstrom einer Hydroxylaminanlage eingesetzt, bei der beispielsweise zunächst Ammoniak mit Luft oder Sauerstoff zu NO oxidiert wird, wobei kleine Mengen an Distickstoffmonoxid als Nebenprodukt gebildet werden. Das NO wird anschließend mit Wasserstoff zu Hydroxylamin hydriert. Nachdem Distickstoffmonoxid unter den Hydrierbedingungen inert ist, reichert es sich im Wasserstoffkreis an. In bevorzugten Verfahrensführungen enthält der Purge-Strom einer Hydroxylaminanlage Distickstoffmonoxid im Bereich von 9 bis 13 Vol.-% in Wasserstoff. Dieser Purge-Strom kann als solcher zur efindungsgemäßen Reinigung eingesetzt werden. Ebenso ist es möglich, diesen Strom bezüglich des Disfickstoffmonoxid-Gehaltes, wie oben beschrieben, geeignet aufzukonzentrieren.

Demgemäß betrifft die vorliegende Erfindung auch ein wie oben beschriebenes Verfahren, wobei das Gasgemisch enthaltend Distickstoffmonoxid das Abgas einer Adipinsäureanlage und/oder einer Dodecandicarbonsäureanlage und/oder einer Hydroxylaminanlage und/oder einer mit dem Abgas einer Adipinsäureanlage und/oder einer Dodecandicarbonsäureanlage und/oder einer Hydroxylaminanlage betriebenen Salpetersäureanlage ist.

Ebenso kann im Rahmen des erfindungsgemäßen Verfahrens Distickstoffmonoxid zum Einsatz im Verfahren gezielt hergestellt werden. Bevorzugt wird dabei unter anderem die Herstellung über die thermische Zersetzung von NH₄NO₃, wie dies beispielsweise in US 3,656,899 beschrieben ist. Ebenfalls bevorzugt wird weiter die Herstellung über die katalytische Oxidation von Ammoniak, wie dies beispielsweise in US 5,849,257 oder in WO 98/25698 beschrieben ist.

Das durch das erfindungsgemäße Reinigungsverfahren erhaltene Gasgemisch G-2 enthält mindestens 50 Vol.-% N₂O, besonders bevorzugt mindestens 60 Vol.-% N₂O und ganz besonders bevorzugt mindestens 75 Vol.-% N₂O. Üblicherweise enthält das Gasgemisch G-2 bis zu 99 Vol.-% N₂O, insbesondere bis zu 97 Vol.-% N₂O, beispielsweise bis zu 96 Vol.% N₂O, bis zu 95 Vol.-% N₂O, bis zu 94 Vol.-% N₂O, bis zu 93 Vol.-% N₂O, bis zu 92 Vol.-% N₂O, bis zu 91 Vol.-% N₂O, bis zu 90 Vol.-% N₂O oder auch bis zu 85 Vol.-% N₂O.

Gleichzeitig enthält das Gasgemisch G-2 weniger als 1 Vol.-% O₂, insbesondere weniger als 0,5 Vol.-% O₂, weniger als 0,5 Vol.-% NOₓ und weniger als 1 Vol.-% NH₃.

Das durch das erfindungsgemäße Reinigungsverfahren erhaltene Gasgemisch G-2 enthält 5 bis 20 Vol.-% CO₂, besonders bevorzugt 6 bis 18 Vol.-% CO₂ und ganz besonders bevorzugt 8 bis 12 Vol.-% CO₂.

Die N₂O-Konzentrierung erfolgt efindungsgemäß durch eine erste selektive Absorption von N₂O und damit vorzugsweise aufgrund der ähnlichen physikalischen Eigenschaften auch von CO₂ aus dem Gasgemisch G-0 in einem geeigneten organischen Lösungsmittel und anschließender Desorption des Gasgemischs G-1 aus dem beladenen Lösungsmittel gemäß Schritt A1 und A2. Das Gasgemisch G-1 wird erfindungsgemäß für eine weitere Aufkonzentrierung in Wasser gemäß Schritt B1 absorbiert. Bei der Desorption gemäß Schritt B2 wird erfindungsgemäß das Gasgemisch G-2 erhalten.

Geeignete Lösungsmittel für die Absorption gemäß Schritt A1 sind solche, die für N₂O und vorzugsweise auch CO₂ als inerter Komponente eine bessere Löslichkeit aufweisen, als für die unerwünschten Komponenten des eintretenden Eduktgases G-0.

Als organische Lösungsmittel können erfindungsgemäß alle Lösungsmittel eingesetzt werden, bei denen das Verhältnis zwischen N₂O-Löslichkeit (in mol/mol Lösungsmittel) und der Löslichkeit der unerwünschten Nebenkomponenten unter den im Absorber herrschenden Bedingungen (dieses Verhältnis wird im folgenden γ genannt) mindestens 5 beträgt. Dies Verhältnis kann für jede einzelne im Gasgemisch enthaltene Komponente bestimmt werden. Bevorzugte organische Lösungsmittel weisen beispielsweise bei 30 °C einen Wert γ_{O2} von 6 bis 30, bevorzugt von 9 bis 25 und einen Wert γ_{N2} von größer 10, bevorzugt von größer als 15, insbesondere von größer als 20 auf.

Beispiele für geeignete Lösungsmittel sind beispielsweise aliphatische Kohlenwasserstoffe, bevorzugt mit mindestens 5 C-Atomen, weiter bevorzugt mit mindestens 8 C-Atomen, substituierte oder unsubstituierte aromatische Kohlenwasserstoffe, Ester, Ether, Amide, Lactone, Lactame, Nitrile, Alkylhalogenide, Olefine oder Mischungen dieser Lösungsmittel.

Ganz besonders bevorzugt sind erfindungsgemäß Lösungsmittel, die einen Siedepunkt bei Normaldruck von mindestens 100°C aufweisen, da dadurch die Lösungsmittelverluste sowohl im Abgasstrom des Absorbers wie auch des Desorbers reduziert werden.

Darüber hinaus weisen erfindungsgemäß geeignete Lösungsmittel gleichzeitig eine gute Löslichkeit für Distickstoffmonoxid auf. Die Löslichkeit wird über das Verhältnis zwischen dem Partialdruck von N₂O in der Gasphase und dem Molanteil von N₂O in der Flüssigphase (Henry-Koeffizient, H_{N2o}) angegeben, d.h. ein kleiner Wert bedeutet eine hohe Löslichkeit von Distickstoffmonoxid im Lösungsmittel. Vorzugsweise ist dies Verhältnis für ein insbesondere in der ersten Stufe eingesetztes organisches Lösungsmittel bei 30°C kleiner als 1000, weiter bevorzugt kleiner als 750, besonders bevorzugt kleiner als 500, insbesondere kleiner als 150.

Geeignete Lösungsmittel sind unter anderem N-Methylpyrrolidon, Dimethylformamid, Dimethylsulfoxid, Propylencarbonat, Sulfolan, N,N-Dimethylacetamid oder Cyclopentan. Besonders bevorzugt sind im Rahmen der vorliegenden Erfindung beispielsweise Toluol, Nitrobenzol, 1,2-Dichlorbenzol, Tetradecan, beispielsweise ein technisches Gemisch aus gesättigten Kohlenwasserstoffen mit überwiegend 14 Kohlenstoffatomen, und Phthalsäuredimethylester.

Daher betrifft die vorliegende Erfindung in einer bevorzugten Ausführungsform ein Verfahren zur Reinigung eines Gasgemisches umfassend Distickstoffmonoxid wie oben beschrieben, wobei das organische Lösungsmittel ausgewählt ist aus der Gruppe bestehend aus Toluol, Nitrobenzol, 1,2-Dichlorbenzol, Tetradecan und Phthalsäuredimethylester.

Im Rahmen der vorliegenden Erfindung hat das Gasgemisch G-1 beispielsweise einen Gehalt an N₂O von 50 bis 80 Vol.-%, vorzugsweise von 55 bis 75 Vol.-%, insbesondere von 58 bis 70 Vol.-%, besonders bevorzugt beispielsweise 59 Vol.-%, 60 Vol.-%, 61 Vol.-%, 62 Vol.-%, 63 Vol.-%, 64 Vol.-%, 65 Vol.-%, 66 Vol.-%, 67 Vol.-%, 68 Vol.-% oder 69 Vol.-%.

Das Gasgemisch G-1 hat beispielsweise einen Gehalt an CO₂ von 5 bis 15 Vol.-%, vorzugsweise von 6 bis 12 Vol.-%, besonders bevorzugt beispielsweise 7 Vol.-%, 9 Vol.-%, 10 Vol.-% oder 11 Vol.-%. Gleichzeitig hat das Gasgemisch G-1 beispielsweise einen Gehalt an O₂ von 1,0 bis 3,0 Vol.-%, vorzugsweise von 1,5 bis 2,5 Vol.-%, besonders bevorzugt beispielsweise 1,1 Vol.-%, 1,2 Vol.-%, 1,3 Vol.-%, 1,4 Vol.-%, 1,5 Vol.-%, 1,6 Vol.-%, 1,7 Vol.-%, 1,8 Vol.-%, 1,9 Vol.-%, 2,0 Vol.-%, 2,1 Vol.-%, 2,2 Vol.-%, 2,3 Vol.-% oder 2,4 Vol.-%. Darüber hinaus kann das Gasgemisch G-1 noch 20 bis 40 Vol.-% N₂ enthalten, vorzugsweise 20 bis 35 Vol.-%, sowie weitere Komponenten, beispielsweise Stickoxide oder Lösungsmittelreste. Dabei ergibt die Summe der Komponenten des Gasgemischs G-1 100 Vol.-%.

Gemäß Schritt B1 und B2 erfolgt eine Absorption/Desorption mit Wasser als Lösungsmittel. Wasser weist eine hohe Selektivität für die gewünschten Komponenten, insbesondere Distickstoffmonoxid und Kohlenstoffdioxid, auf. Gleichzeitig ist die absolute Löslichkeit von Distickstoffmonoxid in Wasser ausreichend, um eine weitere Aufkonzentrierung zu erreichen. Dabei hat Wasser als Lösungsmittel den Vorteil, dass auch unter Druck in Gegenwart von konzentriertem Distickstoffmonoxid keine sicherheitstechnischen Probleme auftreten. Gleichzeitig kann keine Kontaminierung des Gasgemischs G-2 mit einem organischen Lösungsmittel auftreten, die zusätzliche Reinigungsschritte nötig machen würden.

Im Rahmen der vorliegenden Erfindung hat das Gasgemisch G-2 beispielsweise einen Gehalt an N₂O von 70 bis 95 Vol.-%, vorzugsweise von 75 bis 90 Vol.-%, insbesondere von 80 bis 85 Vol.-%, besonders bevorzugt beispielsweise 81 Vol.-%, 82 Vol.-%, 83 Vol.-% oder 84 Vol.-%.

Das Gasgemisch G-2 hat beispielsweise einen Gehalt an CO₂ von 1 bis 20 Vol.-%, vorzugsweise von 5 bis 15 Vol.-%, besonders bevorzugt beispielsweise 6 Vol.-%, 7 Vol.-%, 8 Vol.-%, 9 Vol.-%, 10 Vol.-%, 11 Vol.-%, 12 Vol.-%, 13 Vol.-% oder 14 Vol.-%. Gleichzeitig hat das Gasgemisch G-2 beispielsweise einen Gehalt an O₂ von 0,01 bis 5,0 Vol.-%, vorzugsweise von 0,1 bis 2,5 Vol.-%, besonders bevorzugt beispielsweise 0,2 bis 1,0 Vol.-%. Darüber hinaus kann das Gasgemisch G-2 noch 0,1 bis 10 Vol.-% N₂ enthalten, vorzugsweise 0,5 bis 5 Vol.-%, sowie weitere Komponenten, beispielsweise Stickoxide oder Lösungsmittelreste. Dabei ergibt die Summe der Komponenten des Gasgemischs G-2 100 Vol.-%.

Die Absorption gemäß Schritt A1 bzw. B1 des erfindungsgemäßen Verfahrens kann grundsätzlich nach allen dem Fachmann bekannten Verfahren erfolgen. Insbesondere kann die Absorption von N₂O im Lösungsmittel durch Erhöhung des Drucks des Eduktgases oder durch Absenkung der Temperatur des Lösungsmittels oder durch eine Kombination der genannten Maßnahmen herbeigeführt werden. Unter Lösungsmittel werden im Rahmen der vorliegenden Anmeldung sowohl Wasser als auch organische Lösungsmittel verstanden.

Vorzugsweise wird bei der Absorption zunächst das Gasgemisch komprimiert, beispielsweise auf einen Druck von 10 bis 35 bar, bevorzugt von 15 bis 30 bar, vorzugsweise von 16 bis 25 bar. Anschließend wird das komprimierte Gasgemisch vorzugsweise bei diesem Druck in einem organischen Lösungsmittel gemäß Schritt A1 oder in Wasser gemäß Schritt B1 absorbiert.

Daher betrifft eine bevorzugte Ausführungsform der vorliegenden Erfindung ein wie zuvor beschriebenes Reinigungsverfahren, wobei der Druck bei der Absorption gemäß A1 oder B1 in einem Bereich von 10 bis 35 bar liegt.

Die Absorption gemäß Schritt A1 und Schritt B1 erfolgt erfindungsgemäß in Einrichtungen (Absorber) in denen eine Gas-Flüssig-Phasengrenzfläche erzeugt wird, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind.

Die Führung der Phasen im Absorber kann im Gleichstrom, im Gegenstrom oder einer Kombination der genannten erfolgen.

Die Absorption kann erfindungsgemäß ein- oder mehrstufig durchgeführt werden, vorzugsweise einstufig. Dabei wird bei der Absorption vorzugsweise als Absorber eine Einrichtung mit mehreren theoretischen Trennstufen verwendet, insbesondere 2 bis 8 theoretischen Trennstufen, besonders bevorzugt 3 bis 6.

Mögliche Ausführungsformen des Absorbers sind jeweils Kolonnen mit Böden, beispielsweise Glockenböden oder Siebböden, Kolonnen mit strukturierten Einbauten, wie beispielsweise Packungen, Kolonnen mit unstrukturierten Einbauten, wie beispielsweise Füllkörpern, oder Apparate in denen die Flüssigphase dispergiert vorliegt, beispielsweise durch Versprühen in Düsen, oder eine Kombination der genannten.

Die Desorption von N₂O aus dem beladenen Lösungsmittel gemäß Schritt A2 oder B2 des erfindungsgemäßen Verfahrens kann durch Druckabsenkung über dem Lösungsmittel, Temperaturerhöhung des Lösungsmittels oder durch Strippung mit Lösungsmitteldampf oder einer Kombination der genannten herbeigeführt werden.

Die Anforderungen an die Einrichtungen (Desorber) zur Desorption von N₂O aus dem beladenen Lösungsmittel, sowie die Führung der Phasen sind analog zu denen des Absorbers, d.h. geeignet sind Einrichtungen, in denen eine Gas-Flüssig-Phasengrenzfläche erzeugt wird, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind.

Die Desorption kann erfindungsgemäß ein- oder mehrstufig durchgeführt werden.

Mögliche Ausführungsformen des Desorbers sind ein einfacher (Entspannungs-)Behälter und Kolonnen.

Eine bevorzugte Ausführungsform der vorliegenden Erfindung, in der die Absorption und Desorption apparativ vereint sind, ist beispielsweise die Trennwandkolonne. Dabei wird die Absorption und Desorption durch Teniperaturwechsel mehrstufig im Gegenstrom betrieben kombiniert mit einer Strippung mit Lösungsmitteldampf. Dabei kann sowohl gemäß A1 und A2 als auch gemäß B1 und B2 eine apparative Vereinigung der Absorption und Desorption erfolgen, insbesondere in einer Trennwandkolonne.

In einer bevorzugten Ausführungsform betrifft die vorliegende Erfindung daher ein Verfahren wie oben beschrieben, wobei die Schritte A1 und A2 oder die Schritte B1 und B2 oder die Schritte A1 und A2 und die Schritte B1 und B2 in einer Trennwandkolonne durchgeführt werden.

Im Sinne einer besonders bevorzugten Ausführungsform der Erfindung wird gemäß Schritt A1 zunächst das Gasgemisch G-0 enthaltend N₂O unter erhöhtem Druck p_{Abso} in einer im Gegenstrom betriebenen Absorpfionskolonne mit Füllkörperschüttung absorbiert und gemäß Schritt A2 in einen Behälter überführt, in dem das mit N₂O beladene Lösungsmittel auf einen niedrigeren Druck p_{Deso} < p_{Abso} entspannt wird. Der Prozess wird vorzugsweise nahezu isotherm betrieben mit einer Temperaturdifferenz zwischen Absorptions- und Desorptionstemperatur von maximal 20K, vorzugsweise maximal 15K, insbesondere maximal 10 K. Der Absorptionsdruck beträgt hierbei 1 bis 100 bar, bevorzugt 5 bis 65 bar, insbesondere 10 bis 40 bar, bevorzugt 10 bis 35 bar, besonders bevorzugt 15 bis 30 bar, weiter bevorzugt etwa 16 bis 25 bar und der Desorptionsdruck 0,1 bis 2 bar, vorzugsweise 0,5 bis 1,5 bar, besonders bevorzugt 1,0 bis 1,2 bar.

Vorzugsweise wird ebenfalls gemäß Schritt B1 zunächst das Gasgemisch G-1 unter erhöhtem Druck p_{Abso} in einer im Gegenstrom betriebenen Absorptionskolonne mit Füllkörperschüttung absorbiert und gemäß Schritt B2 in einen Behälter überführt, in dem das mit N₂O beladene Wasser auf einen niedrigeren Druck p_{Deso} < p_{Abso} entspannt wird. Der Prozess wird vorzugsweise ebenfalls nahezu isotherm betrieben mit einer Temperaturdifferenz zwischen Absorptions- und Desorptionstemperatur von maximal 20K, vorzugsweise maximal 15K, insbesondere maximal 10 K. Der Absorptionsdruck beträgt hierbei 1 bis 100 bar, bevorzugt 5 bis 65 bar, insbesondere 10 bis 40 bar, bevorzugt 10 bis 35 bar, besonders bevorzugt 16 bis 30 bar, weiter bevorzugt etwa 20 bis 25 bar und der Desorptionsdruck 0,1 bis 2 bar, vorzugsweise 0,5 bis 1,5 bar, besonders bevorzugt 1,0 bis 1,2 bar.

Das erfindungsgemäße Verfahren kann gemäß einer weiteren Ausführungsform weiterhin einen Schritt C umfassen, wobei der Gehalt an Stickoxiden im Gasgemisch auf höchstens 0,5 Vol.-% bezogen auf das Gesamtvolumen des Gasgemisches eingestellt wird.

Daher betrifft die vorliegende Erfindung auch ein wie zuvor beschriebenes Verfahren, wobei das Verfahren zusätzlich den Schritt
- C: Einstellen des Gehalts an Stickoxiden NOₓ im Gasgemisch auf höchstens 0,5 Vol.-%, bezogen auf das Gesamtvolumen des Gasgemisches,
umfasst.

Dabei kann im Rahmen der vorliegenden Erfindung Schritt C vor oder nach den Schritten A1, A2, B1 und B2 durchgeführt werden. Daher betrifft die vorliegende Erfindung in einer Ausführungsform ein Verfahren wie oben beschrieben, wobei die Schritte A1, A2, B1 und B2 vor dem Schritt C ausgeführt werden. In einer alternativen Ausführungsform betrifft die vorliegende Erfindung ein Verfahren wie oben beschrieben, wobei der Schritt C vor den Schritte A1, A2, B1 und B2 ausgeführt wird.

Sofern Schritt C vor den Schritten A1, A2, B1 und B2 durchgeführt wird, wird der NOₓ Gehalt im Gasgemisch G-0 eingestellt.

Das Gasgemisch G-0 weist in diesem Fall bei der Absorption gemäß A1 vorzugsweise einen N₂O Gehalt von 4 bis 25 Vol.-%, vorzugsweise von 6 bis 20 Vol.-%, insbesondere von 8 bis 18 Vol.-%, besonders bevorzugt beispielsweise 9 Vol.-%, 10 Vol.-%, 11 Vol.-%, 12 Vol.-%, 13 Vol.-%, 14 Vol.-%, 15 Vol.-%, 16 Vol.-% oder 17 Vol.-% auf.

Das Gasgemisch hat beispielsweise einen Gehalt an CO₂ von 0,1 bis 7,5 Vol.-%, vorzugsweise von 0,5 bis 5 Vol.-%, besonders bevorzugt 1 bis 2,5 Vol.-%. Gleichzeitig hat das Gasgemisch beispielsweise einen Gehalt an O₂ von 1 bis 10 Vol.-%, vorzugsweise von 2 bis 7,5 Vol.-%, besonders bevorzugt beispielsweise 3,0 bis 6 Vol.-%. Darüber hinaus kann das Gasgemisch noch 50 bis 95 Vol.-% N₂ enthalten, vorzugsweise 60 bis 90 Vol.-%, besonders bevorzugt 70 bis 85 Vol.-%, sowie weitere Komponenten, beispielsweise Stickoxide oder Lösungsmittelreste. NOₓ kann dabei beispielsweise in einer Menge von 0 bis 0,1 Vol.-% enthalten sein, vorzugsweise 0,0001 bis 0,01 Vol.-%, besonders bevorzugt 0,0005 bis 0,05 Vol.%. Dabei ergibt die Summe der Komponenten des Gasgemischs 100 Vol.-%.

Nach der ersten Absorption/Desorption gemäß A1 und A2 und vor der zweiten Absorption/Desorption gemäß B1 und B2 weist das Gasgemisch vorzugsweise einen N₂O Gehalt von 40 bis 80 Vol.-%, vorzugsweise von 50 bis 75 Vol.-%, insbesondere von 55 bis 70 Vol.-%, besonders bevorzugt beispielsweise 56 Vol.%, 57 Vor.-%, 58 Vol.-%, 59 Vor.-%, 60 Vor.-%, 61 Vol.-%, 62 Vol.-%, 63 Vor.-%, 64 Vor.-%, 65 Vol.-%, 66 Vol.-%, 67 Vol.-%, 68 Vol.-% oder 69 Vol.-% auf.

Das Gasgemisch hat beispielsweise einen Gehalt an CO₂ von 1 bis 15 Vol.-%, vorzugsweise von 2 bis 10 Vol.-%, besonders bevorzugt 7 bis 9 Vol.-%. Gleichzeitig hat das Gasgemisch beispielsweise einen Gehalt an O₂ von 0,5 bis 7,5 Vol.-%, vorzugsweise von 1 bis 5 Vol.-%, besonders bevorzugt beispielsweise 2,5 bis 3,5 Vol.-%. Darüber hinaus kann das Gasgemisch noch 5 bis 40 Vol.-% N₂ enthalten, vorzugsweise 10 bis 35 Vol.-%, besonders bevorzugt 20 bis 30 Vol.-% sowie weitere Komponenten, beispielsweise Stickoxide oder Lösungsmittelreste. NOₓ kann dabei beispielsweise in einer Menge von 0 bis 0,1 Vol.-% enthalten sein, vorzugsweise 0,0001 bis 0,01 Vol.-%, besonders bevorzugt 0,0005 bis 0,05 Vol.-%. Dabei ergibt die Summe der Komponenten des Gasgemischs 100 Vol.-%.

Nach der zweiten Absorption/Desorption gemäß B1 und B2 weist das Gasgemisch vorzugsweise einen N₂O Gehalt von 60 bis 95 Vor.-%, vorzugsweise von 70 bis 90 Vol.-%, insbesondere von 75 bis 85 Vol.-%, besonders bevorzugt beispielsweise 76 Vol.-%, 77 Vol.-%, 78 Vor.-%, 79 Vol.-%, 80 Vol.-%, 81 Vor.-%, 82 Vol.-%, 83 Vor.-%, 84 Vol.-% oder 85 Vol.-% auf.

Das Gasgemisch hat beispielsweise einen Gehalt an CO₂ von 1 bis 20 Vol.-%, vorzugsweise von 5 bis 15 Vol.-%, besonders bevorzugt 7,5 bis 12,5 Vol.-%. Gleichzeitig hat das Gasgemisch beispielsweise einen Gehalt an O₂ von 0,01 bis 7,5 Vol.-%, vorzugsweise von 0,1 bis 5 Vol.-%, besonders bevorzugt beispielsweise 0,2 bis 2,5 Vol.%. Darüber hinaus kann das Gasgemisch noch 0,1 bis 10 Vol.-% N₂ enthalten, vorzugsweise 0,5 bis 5 Vol.-%, besonders bevorzugt 1 bis 2,5 Vol.-%, sowie weitere Komponenten, beispielsweise Stickoxide oder Lösungsmittelreste. NOₓ kann dabei beispielsweise in einer Menge von 0 bis 0,1 Vol.-% enthalten sein, vorzugsweise 0,0001 bis 0,01 Vol.-%, besonders bevorzugt 0,0005 bis 0,05 Vol.-%. Dabei ergibt die Summe der Komponenten des Gasgemischs 100 Vol.-%.

Sofern Schritt C nach den Schritten A1, A2, B1 und B2 durchgeführt wird, wird das Gasgemisch G-2 behandelt.

Nach der zweiten Absorption/Desorption gemäß B1 und B2 und anschließendem Schritt C weist das Gasgemisch vorzugsweise einen N₂O Gehalt von 60 bis 95 Vol.-%, vorzugsweise von 70 bis 90 Vol.-%, insbesondere von 75 bis 85 Vol.-%, besonders bevorzugt beispielsweise 76 Vol.-%, 77 Vol.-%, 78 Vol.-%, 79 Vol.-%, 80 Vol.-%, 81 Vol.-%, 82 Vol.-%, 83 Vol.-%, 84 Vol.-% oder 85 Vol.-% auf.

Das Gasgemisch hat beispielsweise einen Gehalt an CO₂ von 1 bis 20 Vol.-%, vorzugsweise von 5 bis 15 Vol.-%, besonders bevorzugt 7,5 bis 12,5 Vol.-%. Gleichzeitig hat das Gasgemisch beispielsweise einen Gehalt an O₂ von 0,01 bis 7,5 Vol.-%, vorzugsweise von 0,1 bis 5 Vol.-%, besonders bevorzugt beispielsweise 0,2 bis 2,5 Vol.-%. Darüber hinaus kann das Gasgemisch noch 0,1 bis 10 Vol.-% N₂ enthalten, vorzugsweise 0,5 bis 5 Vol.-%, besonders bevorzugt 1 bis 2,5 Vol.-%, sowie weitere Komponenten, beispielsweise Stickoxide oder Lösungsmittelreste. NOₓ kann dabei beispielsweise in einer Menge von 0 bis 0,1 Vol.-% enthalten sein, vorzugsweise 0,0001 bis 0,01 Vol.-%, besonders bevorzugt 0,0005 bis 0,05 Vol.-%. Dabei ergibt die Summe der Komponenten des Gasgemischs 100 Vol.-%.

Im Rahmen der vorliegenden Erfindung kann der Schritt C auch zwischen den Schritten A1 und A2 und den Schritten B1 und B2 durchgeführt werden. In diesem Fall weist das Gasgemisch G-2 annähernd dieselbe Zusammensetzung auf wie das Gasgemisch, das nach der zweiten Absorption/Desorption gemäß B1 und B2 und anschließendem Schritt C erhalten wird.

Ebenso ist es im Rahmen der vorliegenden Erfindung möglich, dass das Verfahren mehrere Schritte C umfasst. Damit kann beispielsweise auch ein Schritt C vor den Schritten A1, A2, B1 und B2 durchgeführt werden und ein weiterer Schritt C nach den Schritten A1, A2, B1 und B2.

Demgemäß betrifft die vorliegende Erfindung auch ein wie zuvor beschriebenes Verfahren, wobei der Schritt C vor den Schritten A1, A2, B1 und B2 ausgeführt wird.

Grundsätzlich kommen für den Schritt C des erfindungsgemäßen Verfahrens alle geeigneten Verfahren zur Abtrennung von NOₓ in Frage. Geeignet sind beispielsweise die katalytische Reduktion mit Kohlenwasserstoffen oder Ammoniak, die katalytische Zersetzung an geeigneten Katalysatoren, Absorption in stark oxidierenden Lösungen sowie die Absorption in sauren oder alkalischen Lösungen.

Geeignete oxidierende Lösungen im Rahmen der vorliegenden Erfindung sind beispielsweise Lösungen von Wasserstoffperoxid. Als stark saure Lösungen sind erfindungsgemäß beispielsweise Lösungen enthaltend Salpetersäure oder Schwefelsäure geeignet. Als alkalische Lösungen eignen sich erfindungsgemäß beispielsweise Lösungen enthaltend Hydroxide oder Carbonate, beispielsweise Natriumhydroxid oder Natriumcarbonat. Als Flüssigkeiten für diese Wäsche eignen sich neben den bereits genannten insbesondere diejenige, die dem Fachmann zur Entfernung von NOₓ aus Abgasen geläufig sind. Als Waschlösungen oder - suspensionen sind beispielsweise geeignet wässrige Lösungen oder Suspensionen enthaltend Magnesiumcarbonat, Magnesiumhydroxid, Lösungen von Vanadium in salpetriger Säure, Ammoniumsulfid und Ammoniumbisulfid, Kalkwasser, Ammoniak, Wasserstoffperoxid und insbesondere Lösungen enthaltend Natriumcarbonat, Natriumbicarbonat oder Natriumhydroxid.

Geeignete Verfahren sind beispielsweise genannt in M. Thiemann et. al in Ullmann's Encyclopedia, 6th Edition, 2000, Electronic Edition, Kapitel "Nitric Acid, Nitrous Acid, and Nitrogen Oxides", Abschnitt 1.4.2.3.

Allgemein erfolgt die NOₓ-Absorption in Einrichtungen in denen eine Gas-Flüssig-Phasengrenzfläche vorliegt, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind. Die Führung der Phasen im Absorber kann im Gleichstrom, im Gegenstrom oder einer Kombination der genannten erfolgen.

Die Absorption kann erfindungsgemäß ein- oder mehrstufig durchgeführt werden.

Die Absorption erfolgt erfindungsgemäß bei Temperaturen zwischen -20 und 100 °C. bevorzugt zwischen 0 und 60 °C, besonders bevorzugt zwischen 0 und 40 °C und bei Drücken zwischen 0,1 und 100 bar, bevorzugt zwischen 1 und 30 bar.

Mögliche Ausführungsformen des Absorbers sind Kolonnen mit Böden, beispielsweise Glockenböden oder Siebböden, Kolonnen mit strukturierten Einbauten wie beispielsweise Packungen, Kolonnen mit unstrukturierten Einbauten wie beispielsweise Füllkörpern, oder Apparate in denen die Flüssigphase dispergiert vorliegt, beispielsweise durch Versprühen in Düsen, oder eine Kombination der genannten.

In einer weiteren Ausführungsform betrifft die vorliegende Erfindung daher ein Verfahren wie oben beschrieben, wobei Schritt C die Absorption von Stickoxiden in saurer oder alkalischer Lösung umfasst.

Im Sinne der vorliegenden Erfindung kann die Abtrennung von NOₓ durch Absorption in einer sauren oder einer alkalischen Lösung erfolgen. Die Absorption wird zwischen -20 und 120°C, insbesondere zwischen -10 und 75°C, bevorzugt zwischen 0 und 60°C, beispielsweise zwischen 0 und 40 °C und bei einem Druck zwischen 0,2 und 100 bar, insbesondere zwischen 0,5 bis 50 bar, bevorzugt zwischen 1 und 10 bar durchgeführt.

Sofern die NOₓ-Konzentration im N₂O-haltigen Gasgemisch mehr als 1 Vol.-% beträgt, wird als Lösungsmittel für Schritt C bevorzugt wässrige Salpetersäure eingesetzt, mit einem HNO₃-Gehalt zwischen 0 und 69 Gew.-%, bevorzugt zwischen 0 und 10 Gew.-%. Vorteilhaft hierbei ist, dass die NOₓ-Abreicherung in der Gasphase mit der Herstellung von Salpetersäure mit 1 bis 69 Gew.-% HNO₃ einhergeht. Im Sinne der weiteren Nutzbarkeit wird dabei bevorzugt eine Salpetersäure mit 30 bis 60 Gew.-% HNO₃ hergestellt.

Diese Vorgehensweise kommt im Rahmen der vorliegenden Erfindung beispielsweise dann bevorzugt zum Einsatz, wenn das N₂O-haltige Eduktgas aus einem Carbonsäureprozess (z.B. Adipinsäure) stammt, wobei NOₓ-Konzentrationen von 1 bis 50 Vol.-% vorliegen. Die NOₓ-Abtrennung gemäß Schritt C wird in diesem Fall bevorzugt vor der N₂O-Aufkonzentrierung gemäß Schritt A1 und A2 durchgeführt.

Gemäß einer Ausführungsform der vorliegenden Erfindung kann an die Schritte A1, A2, B1 und B2 ein weiterer Schritt C anschließen, vorzugsweise eine chemische Wäsche, besonders bevorzugt mit Natriumcarbonat-Lösung oder Natronlauge.

Im Rahmen der vorliegenden Erfindung kann der NOₓ Gehalt vorzugsweise durch eine selektive katalytische Reduktion mit Ammoniak eingestellt, bei der sich N₂O inert verhält. Diese sogenannte SCR-DeNOx- oder DeNOx-Technologie wird beispielsweise in Ullmann's Encyclopedia of Chemical Technology, Kapitel "Air", Abschnitt 7.2.3.1. "Catalytic Reduction of Nitrogen Oxides in Flue Gases and Process Off-Gases", von J. Wolf et al., 6. Ausgabe (Online Edition), 2000. beschrieben. Gemäß dieser bevorzugten Ausführungsform der vorliegenden Erfindung können NOₓ Konzentrationen von weniger als 100 ppm, vorzugsweise weniger als 50 ppm, beispielsweise weniger als 25 ppm und besonders bevorzugt von bis zu 5 ppm und sehr niedrige NH₃-Konzentrationen im Produkt erreicht werden, beispielsweise weniger als 10 ppm.

Gemäß einer bevorzugten Ausführungsform wird als Schritt C die katalytische Reduktion mit Ammoniak vor den Schritten A1, A2, B1 und B2 durchgeführt.

Gemäß einer besonders bevorzugten Ausführungsform der vorliegenden Erfindung kann das in das erfindungsgemäße Verfahren eingesetzte Gasgemisch enthaltend Distickstoffmonoxid aus einer Adipinsäureanlage stammen. Vorzugsweise wird das Abgas der Adipinsäure-Anlage mit NO-Synthesegas gemischt und abgekühlt. Der gasförmiger Strom wird dann komprimiert, vorzugsweise auf 7 bar, und gegebenenfalls mit Luft versetzt. Das heiße Gas nach der Kompression wird abgekühlt und in einen Absorptionsturm geleitet, in dem NOₓ abgereichert wird. Das Gas am Kopf der Kolonne hat vorzugsweise eine Temperatur von etwa 0 bis 100°C, beispielsweise 20 bis 50°C, vorzugsweise 30 bis 45°C, insbesondere 35 bis 40°C bei einem Druck von 1 bis 15 bar, bevorzugt 4 bis 14 bar, besonders bevorzugt 5 bis 13 bar, insbesondere 6 bis 12 bar.

Das Abgas kann direkt in das erfindungsgemäße Verfahren eingesetzt werden. Gemäß einer weiter bevorzugten Ausführungsform kann jedoch das Abgas auf 100 bis 250°C, vorzugsweise 150 bis 200 °C, bevorzugt auf 160 bis 180°C, besonders bevorzugt auf 170°C aufgeheizt und zur Umsetzung gemäß Schritt C in die DeNOx-Anlage geführt werden.

Anschließend wird der Strom vorzugsweise abgekühlt, komprimiert und wieder abgekühlt, bevor eine Absorption/Desorption gemäß Schritt A1 und A2 und B1 und B2 durchgeführt wird.

Sofern eine NOₓ-Konzentration von < 1 Vol.-% im N₂O-haltigen Gasgemisch vorliegt, wie beispielsweise bei einem Abgas einer Salpetersäureanlage, wird als Absorbens für Schritt C bevorzugt eine alkalische Lösung eingesetzt. Im Sinne der vorliegenden Erfindung kommt diese Vorgehensweise bevorzugt zur Feinreinigung des N₂O-Gases nach der Aufkonzentrierung gemäß Schritt A1, A2, B1 und B2 zum Einsatz.

Neben den Schritten A1, A2, B1 und B2 oder gegebenenfalls C kann das erfindungsgemäße Verfahren auch weitere Schritte umfassen. So kann das Verfahren beispielsweise auch eine weitere Behandlung zwischen den Schritten A und B und dem Schritt C umfassen. Derartige Behandlungen umfassen beispielsweise eine Änderung der Temperatur oder eine Änderung des Drucks oder eine Änderung der Temperatur und des Drucks.

Dabei kann sich beispielsweise die Zusammensetzung eines Gasgemischs ändern, beispielsweise durch Kondensation einer der Komponenten. Bei diesen Komponenten kann es sich beispielsweise um Wasser oder ein anderes Lösungsmittel handeln, vorzugsweise um ein Lösungsmittel, das im Rahmen des erfindungsgemäßen Verfahrens für die Absorption gemäß Schritt A1 eingesetzt wird.

Erfindungsgemäß ist es möglich, dass aus dem Gasgemisch weitere Komponenten abgetrennt werden. So ist es beispielsweise möglich, dass aus dem Gasgemisch G-2 durch Kompression und anschließendes Abkühlen Spuren von Wasser abgetrennt werden, die nach der Desorption gemäß B2 im Gasgemisch G-2 enthalten sein können.

Dabei wird das Gasgemisch G-2 vorteilhafterweise auf einen Druck von 1 bis 35 bar komprimiert, bevorzugt 2 bis 30 bar, weiter bevorzugt 3 bis 27 bar. Eine Abkühlung erfolgt vorzugsweise anschließend, bevorzugt auf 2 bis 25 °C, besonders bevorzugt 3 bis 20 °C, insbesondere 4 bis 15°C. weiter bevorzugt 5 bis 10°C.

Ebenso ist es im Rahmen der vorliegenden Erfindung möglich, beispielsweise eine Teilkondensation von Distickstoffmonoxid durchzuführen oder eine Rektifikation, insbesondere zur Abtrennung der Leichtsieder wie beispielsweise Sauerstoff und Stickstoff.

Erfindungsgemäß kann das durch das erfindungsgemäße Verfahren gereinigte Gasgemisch G-2 enthaltende Distickstoffmonoxid in einer weiteren Umsetzung eingesetzt werden. Dazu kann das Gasgemisch gasförmig eingesetzt werden. Es ist jedoch auch möglich, das erhaltene Gasgemisch zunächst derart zu behandeln, dass das Gasgemisch in flüssiger oder überkritischer Form vorliegt und dann in einer weiteren Umsetzung eingesetzt wird. Das Gasgemisch kann durch geeignete Wahl des Drucks oder der Temperatur verflüssigt werden.

Damit betrifft die vorliegende Erfindung auch ein Verfahren, wobei das erhaltene Gasgemisch G-2 verflüssigt wird.

Vorzugsweise wird dazu das Gasgemisch G-2 zunächst komprimiert und anschließend gekühlt. Dabei wird das Gasgemisch G-2 vorteilhafterweise auf einen Druck von 1 bis 35 bar komprimiert, bevorzugt 2 bis 30 bar, weiter bevorzugt 3 bis 27 bar. Eine Abkühlung erfolgt vorzugsweise anschließend, bevorzugt auf 10 bis -70 °C, besonders bevorzugt 8 bis -30 °C, insbesondere 5 bis -20°C.

Das nach einem erfindungsgemäßen Verfahren erhaltene Gasgemisch G-2 enthaltend Distickstoffmonoxid kann grundsätzlich für alle Anwendungen eingesetzt werden, bei denen üblicherweise reine Distickstoffmonoxidströme oder mit Inertgas versetzte Distickstoffmonoxidströme eingesetzt werden. Insbesondere eignen sich die Gasgemische beispielsweise für die Oxidation von Methanol zu Formaldehyd wie beispielsweise in der EP-A 0 624 565 oder DE-A 196 05 211 beschrieben.

Durch das erfindungsgemäße Verfahren werden Gasgemische G-2 enthaltend Distickstoffmonoxid erhalten, die einen besonders geringen Anteil an störenden Nebenkomponenten enthalten. Dies ist insbesondere für die Verwendung des Gasgemischs enthaltend Distickstoffmonoxid als Oxidationsmittel vorteilhaft, da durch den geringen Anteil an störenden Nebenkomponenten kaum Nebenreaktionen auftreten und somit besonders reine Produkte erhalten werden können. Vorzugsweise enthält das Gasgemisch G-2 nach der erfindungsgemäßen Reinigung neben Distickstoffmonoxid auch Kohlenstoffdioxid.

Das erfindungsgemäß gereinigte Gasgemisch G-2 enthält vorzugsweise 50 bis 99,9 Vol.-% Distickstoffmonoxid, 0,1 bis 25 Vol.-% Kohlenstoffdioxid und 0 bis 25 Vol.-% weitere Gase. Die angegebenen Vol.-% beziehen sich jeweils auf das gesamte Gasgemisch G-2. Die Summe der einzelnen Komponenten des Gasgemischs G-2 ergibt dabei immer 100 Vol.-%.

Vorzugsweise enthält das erfindungsgemäß gereinigte Gasgemisch G-2 65 bis 95 Vol.-% Distickstoffmonoxid, insbesondere 75 bis 92,5 Vol.%, besonders bevorzugt 85 bis 90 Vol.-% Distickstoffmonoxid.

Das erfindungsgemäß gereinigte Gasgemisch G-2 enthält weiterhin 0,1 bis 25 Vol.-% Kohlenstoffdioxid. Vorzugsweise enthält das Gasgemisch G-2 1 bis 20 Vol.-% Kohlenstoffdioxid, insbesondere 2 bis 15 Vol.-%, besonders bevorzugt 5 bis 13 Vol.-% Kohlenstoffdioxid.

Vorzugsweise enthält das Gasgemisch G-2 0,01 bis 20 Vol.-% weitere Gase, beispielsweise 0,1 bis 15 Vol.-% , insbesondere 0,5 bis 10 Vol.-%, besonders bevorzugt 1 bis 5 Vol.-% weitere Gase. Das erfindungsgemäß gereinigte Gasgemisch G-2 kann ein oder mehrere weitere Gase enthalten, wobei die angegebene Menge auf die Summe der enthaltenen Gase bezogen ist.

Es wurde gefunden, dass bei der Anwesenheit von CO₂ als Inertgas in Gasgemischen enthaltend N₂O im Vergleich zu anderen Inertgasen deutlich geringere Mengen Kohlenstoffdioxid benötigt werden, um einen sicheren Betrieb zu gewährleisten, beispielsweise um einen Selbstzerfall von Distickstoffmonoxid zu unterbinden.

Das erfindungsgemäße Verfahren hat unter anderem den Vorteil, dass CO₂ neben der im Vergleich zu anderen Inertgasen guten Inertisierungswirkung eine dem N₂O sehr ähnliche Siedekurve und ähnliche kritische Parameter aufweist. Dadurch lässt sich das im erfindungsgemäßen Verfahren erhaltene Gasgemisch G-2 enthaltend N₂O und gegebenenfalls CO₂ bei einer höheren Temperatur kondensieren, als ein vergleichbares Gemisch aus N₂O und einem anderen Inertgas. Durch die sehr ähnlichen Siedekurven hat das kondensierte Gasgemisch fast die gleiche Zusammensetzung wie das gasförmige Gemisch, so dass das Inertisierungsmittel in der flüssigen Phase erhalten bleibt.

Darüber hinaus weist CO₂ eine gute Löslichkeit in organischen Verbindungen auf, so dass ein geringerer Druck ausreicht, um bei einer Reaktion in flüssiger Phase die Bildung einer Gasphase im Reaktor zu vermeiden.

Ein Gasgemisch erhältlich nach einem erfindungsgemäßen Verfahren wie oben beschrieben kann als Oxidationsmittel, insbesondere als Oxidationsmittel für Olefine, verwendet werden.

Grundsätzlich eignen sich die erfindungsgemäß erhältlichen Gasgemische enthaltend Distickstoffmonoxid zur Oxidation von Olefinen. Geeignete Olefine sind beispielsweise offenkettige oder cyclische Olefine mit einer oder mehreren Doppelbindungen. Weiter bevorzugt sind cyclische Olefine mit einer oder mehreren Doppelbindungen, beispielsweise Cyclopenten, Cyclohexen, Cyclohepten, Cycloocten, Cyclodecen, Cycloundecen, Cyclododecen, 1,4-Cyclohexadien, 1,5-Cyclooctadien, 1,5-Cyclododecadien oder 1,5,9-Cyclododecatrien.

Der angereicherte und gereinigte N₂O-haltige Gasstrom G-2 eignet sich ganz besonders zur Oxidation von Olefinen zu Ketonen. Für diesen Zweck kann vorzugsweise entweder das gasförmige N₂O direkt in das zu oxidierende Olefin oder ein anderes Lösungsmittel absorbiert werden oder das N₂O kann zuerst verflüssigt werden bevor es mit dem Olefin zur Reaktion gebracht wird.

Insbesondere beim Einsatz eines verflüssigten Gasgemischs enthaltend Distickstoffmonoxid ist es vorteilhaft, wenn der Anteil an Inertgasen im Gasgemisch möglichst gering ist, da ansonsten das Reaktorvolumen unnötig vergrößert wird.

Für die Verwendung als Oxidationsmittel, insbesondere für Olefine, kann die Oxidation generell gemäß sämtlicher Verfahrensführungen erfolgen, bei denen die Oxidation, insbesondere des Olefins, stattfindet. Insbesondere sind sowohl kontinuierliche Verfahrensführungen und Fahrweisen der Umsetzung als auch Batch-Reaktion möglich. Die Reaktionsbedingungen für die Oxidation werden erfindungsgemäß so gewählt, dass eine Reaktion stattfindet. Druck und Temperatur können entsprechend gewählt werden.

Vorzugsweise liegt der Druck in einem Bereich bis 350 bar, beispielsweise 1 bis 320 bar, bevorzugt 2 bis 300 bar, insbesondere 3 bis 280 bar. Die Temperatur liegt vorzugsweise in einem Bereich von 220 bis 320°C, beispielsweise 230 bis 300°C, insbesondere 250 bis 290°C.

Dabei kann die Oxidation in Gegenwart eines geeigneten Lösungsmittels durchgeführt werden. Es ist aber ebenso möglich, die Oxidation ohne den Zusatz eines Lösungsmittels durchzuführen.

Es ist jedoch im Rahmen der vorliegenden Erfindung auch möglich, dass das Gasgemisch G-2 durch geeignete Wahl des Drucks und/oder der Temperatur verflüssigt wird oder in einen überkritischen Zustand gebracht wird. Das verflüssigte Gasgemisch kann dann direkt in die Oxidation eingesetzt werden.

Vorzugsweise wird die Oxidation in diesem Fall durch geeignete Wahl des Drucks und der Temperatur so geführt, dass in der Reaktionszone keine Gasphase auftritt.

Vorzugsweise liegt der Druck in einem Bereich bis 350 bar, beispielsweise von 1 bis 320 bar, bevorzugt von 2 bis 300 bar, insbesondere von 3 bis 280 bar. Die Temperatur liegt vorzugsweise in einem Bereich von 220 bis 320°C, beispielsweise 230 bis 300°C, insbesondere 250 bis 290°C.

Daher betrifft die vorliegende Erfindung auch ein Verfahren zur Herstellung eines Ketons, mindestens umfassend die folgenden Schritte
- A1: Absorption eines Gasgemisches G-0 enthaltend Distickstoffmonoxid in einem organischen Lösungsmittel
- A2: Desorption eines Gasgemisches G-1 aus dem beladenen organischen Lösungsmittel
- B1: Absorption des Gasgemisches G-1 in Wasser
- B2: Desorption eines Gasgemisches G-2 aus dem beladenen Wasser
- D: Inkontaktbringen des Gasgemisches G-2 mit mindestens einem Olefin.

Das Verfahren kann wie zuvor beschrieben auch mindestens einen Schritt C umfassen.

Für die bevorzugten Ausführungsformen der Schritte A1, A2 und B1 und B2 gelten die vorstehenden Ausführungen. Auch für die Herstellung eines Ketons kann dabei die Reihenfolge der Schritte A1, A2, B1 und B2 einerseits und C andererseits variieren. Erfindungsgemäß kann dabei der Schritt C nach den Schritten A1, A2, B1 und B2 ausgeführt werden. Es ist jedoch ebenso möglich, dass der Schritt C vor den Schritten A1, A2, B1 und B2 ausgeführt wird. In jedem Fall wird jedoch der Schritt D nach den Schritten A1, A2, B1 und B2 ausgeführt.

Grundsätzlich ist es möglich, den Schritt C vor den Schritten A1, A2, B1 und B2 auszuführen, Ebenso ist es jedoch im Rahmen der vorliegenden Erfindung möglich, dass der Schritt C nach den Schritten A1, A2, B1 und B2 ausgeführt wird. Daher betrifft die vorliegende Erfindung in einer weiteren Ausführungsform auch ein Verfahren zur Herstellung eines Ketons wie oben beschrieben, bei dem die Schritte A1, A2, B1 und B2 vor dem Schritt C ausgeführt werden. Gemäß einer alternativen Ausführungsform betrifft die vorliegende Erfindung auch ein Verfahren zur Herstellung eines Ketons, bei dem der Schritt C vor den Schritte A1, A2, B1 und B2 ausgeführt wird.

Ebenso ist es im Rahmen der vorliegenden Erfindung möglich, dass das Verfahren mehrere Schritte A1 und A2 oder mehrere Schritte B1 und B2 oder mehrere Schritte C umfasst, wobei der Schritt C vor oder nach den Schritten A1, A2, B1 und B2 durchgeführt werden kann.

Die Umsetzung gemäß Schritt D kann generell gemäß sämtlicher Verfahrensführungen erfolgen, bei denen das Olefin und das Gasgemisch G-2 enthaltend Distickstoffmonoxid miteinander reagieren. Insbesondere sind sowohl kontinuierliche Verfahrensführungen und Fahrweisen der Umsetzung als auch Batch-Reaktion möglich. Die Reaktionsbedingungen für Schritt D werden erfindungsgemäß so gewählt, dass eine Reaktion des mindestens einen Olefins mit dem erfindungsgemäß gereinigten Gasgemisch stattfindet. Druck und Temperatur können entsprechend gewählt werden.

Dabei kann die Reaktion in Gegenwart eines geeigneten Lösungsmittels durchgeführt werden. Es ist erfindungsgemäß aber ebenso möglich, die Umsetzung gemäß Schritt D ohne den Zusatz eines Lösungsmittels durchzuführen.

Efindungsgemäß ist es jedoch auch möglich, dass das Verfahren zur Herstellung eines Ketons weitere Schritte umfasst. So kann das Gasgemisch enthaltend Distickstoffmonoxid beispielsweise vor Schritt D und nach den Schritten A1, A2 und B1 und B2 behandelt werden. Eine mögliche Behandlung ist beispielsweise eine Änderung von Druck und/oder Temperatur des Gasgemischs. Eine weitere mögliche Behandlung ist beispielsweise die Absorption in einem Lösungsmittel, so dass das absorbierte Gasgemisch in Schritt D eingesetzt werden kann. Dabei kann das Lösungsmittel jedes geeignete Lösungsmittel sein. Bevorzugt handelt es sich bei dem Lösungsmittel um das Olefin, das gemäß Schritt D oxidiert werden soll.

Es ist jedoch im Rahmen der vorliegenden Erfindung auch möglich, dass das Gasgemisch G-2 enthaltend Distickstoffmonoxid vor Schritt D und nach den Schritten A1, A2 und B1 und B2 durch geeignete Wahl des Drucks und/oder der Temperatur verflüssigt wird oder in einen überkritischen Zustand gebracht wird. Das verflüssigte Gasgemisch enthaltend Distickstoffmonoxid kann dann gemäß Schritt D direkt mit dem Olefin in Kontakt gebracht werden.

Daher betrifft die vorliegende Erfindung gemäß einer weiteren Ausführungsform auch ein Verfahren zur Herstellung eines Ketons wie oben beschrieben, wobei das in Schritt D eingesetzte Gasgemisch verflüssigte ist.

Grundsätzlich können in Schritt D des erfindungsgemäßen Verfahrens alle geeigneten Olefine eingesetzt werden, beispielsweise Olefine mit 2 bis 18 C-Atomen, insbesondere Olefine mit 5 bis 12 C-Atomen. Geeignete Olefine sind beispielsweise offenkettige oder cyclische Olefine mit einer oder mehreren Doppelbindungen. Weiter bevorzugt sind cyclische Olefinen mit einer oder mehreren Doppelbindungen, beispielsweise Cyclopenten, Cyclohexen, Cyclohepten, Cycloocten, Cyclodecen, Cycloundecen, Cyclododecen, 1,4-Cyclohexadien, 1,5-Cyclooctadien, 1,5-Cyclododecadien oder 1,5,9-Cyclododecatrien.

Besonders bevorzugt wird als Olefin Cyclopenten, Cyclododecen oder 1,5,9-Cyclododecatrien eingesetzt. Daher betrifft die vorliegende Erfindung in einer bevorzugten Ausführungsform ein Verfahren zur Herstellung eines Ketons wie oben beschrieben, wobei das Olefin ausgewählt ist aus der Gruppe bestehend aus Cyclopenten, Cyclododecen und 1,5,9-Cyclododecatrien.

Im Folgenden wird die Erfindung anhand von Beispielen näher erläutert.

### BEISPIELE

### Beispiel 1 (Vergleichsbeispiel, zweifache Absorption/Desorption in Nitrobenzol):

Das Abgas einer Salpetersäureanlage, die mit Abgas aus einer Adipinsäureanlage betrieben wird und in etwa 1500 Vol.-ppm NOₓ enthält, wird zunächst in einer DeNOx-Stufe entstickt.

Das so erhaltene Gasgemisch G-0 (2 kg/h) wird auf 25 bar komprimiert und bei 35 °C in einer Absorptionskolonne A1 (Durchmesser= 80 mm, Höhe= 1800 mm, mit einer Maschendraht-Packung (Kühni Rombopak 9M) gefüllt) in Nitrobenzol absorbiert. Das mit N₂O beladene Nitrobenzol aus A1 wird dann in einem Flashtopf A2 ebenfalls bei 35°C auf 1,1 bar entspannt. Im Kreislauf über A1 und A2 werden 200 kg/h Nitrobenzol umgewälzt.

Das aus dem ersten Desorber erhaltene Gasgemisch G-1 wird erneut auf 25 bar komprimiert und bei 35 °C in einer zweiten Absorptionskolonne B1 (Durchmesser= 30 mm, Höhe= 1800 mm, mit einer Maschendraht-Packung (Kühni Rombopak 9M) gefüllt) in Nitrobenzol absorbiert. Das mit N₂O beladene Nitrobenzol aus B1 wird dann einem Flashtopf B2 ebenfalls bei 35°C auf 1,1 bar entspannt. Daraus resultiert Gasgemisch G-2. Im Kreislauf über B1 und B2 werden 25 kg/h Nitrobenzol umgewälzt.

| Komponente | G-0, [Vol.-%] | G-1, [Vol.-%] | G-2, [Vol.-%] |
|---|---|---|---|
| N₂O | 8,1 | 57,2 | 86,5 |
| N₂ | 86,5 | 30,2 | 1,9 |
| CO₂ | 1,1 | 7,1 | 10,5 |
| H₂O | 0,3 | 2,6 | 0,7 |
| O₂ | 3,1 | 1,9 | 0,2 |
| NOₓ | 13 vppm | 32 vppm | 50 vppm |

Wegen der hohen Konzentration von N₂O in der Gasphase in den Apparaten B1 und B2 und der Anwesenheit des brennbaren organischen Lösungsmittels Nitrobenzol sind diese Apparate sicherheitstechnisch kritisch. Um einen sicheren Betrieb der Stufen B1 und B2 zu gewährleisten müssten diese, um nicht auszuschließende Zündung unbeschadet zu überstehen, druckstoßfest bei 1000 bar ausgelegt werden.

### Beispiel 2 (Absorption/Desorption in Nitrobenzol und Absorption/Desorption in Wasser):

Das Abgas einer Salpetersäureanlage, die mit Abgas aus einer Adipinsäureanlage betrieben wird und in etwa 1500 Vol.-ppm NOₓ enthält, wird zunächst in einer DeNOₓ-Stufe entstickt.

Das so erhaltene Gasgemisch G-0 (2 kg/h) wird auf 25 bar komprimiert und bei 35 °C in der gleichen Absorptionskolonne wie im Beispiel 1 in Nitrobenzol absorbiert. Die Desorption erfolgt wie oben bei 1,1 bar und 35 °C, um ein identisches Gasgemisch G-1 zu generieren.

Das Gasgemisch G-1 wird wie oben auf 25 bar komprimiert und bei 35 °C in einer Absorptionskolonne B1 (Durchmesser= 70 mm, Höhe= 1800 mm, mit einer Maschendrahtpackung gefüllt) in Wasser absorbiert. Im Kreislauf über B1 und B2 werden dabei 112 kg/h Wasser umgewälzt. Das mit N₂O beladene Wasser aus B1 wird dann in einem Flashtopf B2 ebenfalls bei 35°C auf 1,1 bar entspannt. Daraus resultiert Gasgemisch G-2.

| Komponente | G-0, [Vol.-%] | G-1, [Vol.-%] | G-2, [Vol.-%] |
|---|---|---|---|
| N₂O | 8,1 | 58,0 | 81,6 |
| N₂ | 86,5 | 29,3 | 2,0 |
| CO₂ | 1,1 | 7,3 | 10,7 |
| H₂O | 0,3 | 2,4 | 5,3 |
| O₂ | 3,1 | 1,9 | 0,3 |
| NOₓ | 13 vppm | 21 vppm | 30 vppm |

Wenn man vom Wassergehalt absieht, hat das Gasgemisch G-2 eine sehr ähnliche Zusammensetzung wie im Beispiel 1. Da aber in der zweite Absorptions/Desorptions-Stufe kein organisches Lösungsmittel vorhanden ist, sind die Stufen B1 und B2 sicherheitstechnisch leicht beherrschbar. Es reicht aus, die Anlagen auf 30 bar Maximaldruck auszulegen.

Das erfindungsgemäße Verfahren bietet also einen deutlichen wirtschaftlichen Vorteil in Form von niedrigeren Investitionen und geringeren Aufwendungen für Sicherheitstechnik.

## Patentansprüche

1. Verfahren zur Reinigung eines Gasgemisches G-0 enthaltend Distickstoffmonoxid, mindestens umfassend die folgenden Schritte:
A1 Absorption des Gasgemisches G-0 in einem organischen Lösungsmittel
A2 Desorption eines Gasgemisches G-1 aus dem beladenen organischen Lösungsmittel
B1 Absorption des Gasgemisches G-1 in Wasser
B2 Desorption eines Gasgemisches G-2 aus dem beladenen Wasser.

2. Verfahren nach Anspruch 1, wobei das Gasgemisch enthaltend Distickstoffmonoxid das Abgas einer Adipinsäureanlage und/oder einer Dodecandicarbonsäureanlage und/oder einer Hydroxylaminanlage und/oder einer mit dem Abgas einer Adipinsäureanlage und/oder einer Dodecandicarbonsäureanlage und/oder einer Hydroxylaminanlage betriebenen Salpetersäureanlage ist.

3. Verfahren nach einem der Ansprüche 1 oder 2, wobei das organische Lösungsmittel ausgewählt ist aus der Gruppe bestehend aus Toluol, Nitrobenzol, 1,2-Dichlorbenzol, Tetradecan und Phthalsäuredimethylester.

4. Verfahren nach einem der Ansprüche 1 bis 3, wobei der Druck bei der Absorption gemäß A1 oder B1 in einem Bereich von 10 bis 35 bar liegt.

5. Verfahren nach einem der Ansprüche 1 bis 4, wobei die Schritte A1 und A2 oder die Schritte B1 und B2 oder die Schritte A1 und A2 und die Schritte B1 und B2 in einer Trennwandkolonne durchgeführt werden.

6. Verfahren nach einem der Ansprüche 1 bis 5, wobei das Verfahren zusätzlich den Schritt
C Einstellen des Gehalts an Stickoxiden NOₓ im Gasgemisch auf höchstens 0,5 Vol.-%, bezogen auf das Gesamtvolumen des Gasgemisches,
umfasst.

7. Verfahren nach einem der Ansprüche 1 bis 6, wobei der Schritt C vor den Schritten A1, A2, B1 und B2 ausgeführt wird.

8. Verfahren nach einem der Ansprüche 1 bis 7, wobei das erhaltene Gasgemisch G-2 verflüssigt wird.

## Claims

1. A process for purifying a gas mixture G-0 comprising dinitrogen monoxide, said process comprising at least the following steps:
A1 absorption of the gas mixture G-0 in an organic solvent
A2 desorption of a gas mixture G-1 from the laden organic solvent
B1 absorption of the gas mixture G-1 in water
B2 desorption of a gas mixture G-2 from the laden water.

2. The process according to claims 1, wherein the gas mixture comprising dinitrogen monoxide is the offgas of an adipic acid plant and/or of a dodecanedicarboxylic acid plant and/or of a hydroxylamine plant and/or of a nitric acid plant operated with the offgas of an adipic acid plant and/or of a dodecanedicarboxylic acid plant and/or of a hydroxylamine plant.

3. The process according to claim 1 or 2, wherein the organic solvent is selected from the group consisting of toluene, nitrobenzene, 1,2-dichlorobenzene, tetradecane and dimethyl phthalate.

4. The process according to any of claims 1 to 3, wherein the pressure in the absorption according to A1 or B1 is in a range of from 10 to 35 bar.

5. The process according to any of claims 1 to 4, wherein steps A1 and A2 or the steps B1 and B2 or the steps A1 and A2 and the steps B1 and B2 are carried out in a dividing wall column.

6. The process according to any of claims 1 to 5, wherein the process additionally comprises the step
C adjusting the content of nitrogen oxides NOₓ in the gas mixture to at most 0.5 % by volume based on the total volume of the gas mixture.

7. The process according to any of claims 1 to 6, wherein the step C is performed before the steps A1, A2, B1 and B2.

8. The process according to any of claims 1 to 7, wherein the resulting gas mixture G-2 is liquefied.

## Revendications

1. Procédé pour la purification d'un mélange de gaz G-0 contenant de l'oxyde nitreux, comportant au moins les étapes suivantes :
A1 absorption du mélange de gaz G-0 dans un solvant organique
A2 désorption d'un mélange de gaz G-1 du solvant organique chargé
B1 absorption du mélange de gaz G-1 dans de l'eau
B2 désorption d'un mélange de gaz G-2 de l'eau chargée.

2. Procédé selon la revendication 1, dans lequel le mélange de gaz contenant de l'oxyde nitreux est un gaz résiduel d'une unité de production d'acide adipique et/ou d'une unité de production d'acide 1,12-dodécanedioïque et/ou d'une unité de production d'hydroxylamine et/ou un gaz résiduel d'une unité de production d'acide nitrique alimentée avec le gaz résiduel d'une unité de production d'acide adipique et/ou d'une unité de production d'acide 1,12-dodécanedioïque et/ou d'une unité de production d'hydroxylamine.

3. Procédé selon la revendication 1 ou 2, dans lequel le solvant organique est choisi dans le group consistant en le toluène, le nitrobenzène, 1,2-dichlorobenzène, le tétradécane, et le phtalate de diméthyle.

4. Procédé selon l'une des revendications 1 à 3, dans lequel la pression pendant l'absorption selon A1 ou B1 est compris dans la gamme allant de 10 à 35 bar.

5. Procédé selon l'une des revendications 1 à 4, dans lequel les étapes A1 et A2 ou les étapes B1 et B2 ou les étapes A1 et A2 et les étapes B1 et B2 sont effectués dans une colonne à paroi de séparation.

6. Procédé selon l'une des revendications 1 à 5, selon lequel le procédé comprend en outre l'étape
C ajustement de la teneur d'oxydes d'azote NOₓ dans le mélange de gaz à 0,5 % en volume au maximum, par rapport au volume total du mélange de gaz.

7. Procédé selon l'une des revendications 1 à 6, selon lequel l'étape C est effectué avant les étapes A1, A2, B1 et B2.

8. Procédé selon l'une des revendications 1 à 7, selon lequel le mélange de gaz G-2 obtenu est liquéfié.
